# EUROPEAN PATENT SPECIFICATION

(11) **EP 4 114 214 B1**
(45) Date of publication and mention of the grant of the patent: **07.05.2025**
(21) Application number: 21707761.9
(22) Date of filing: 02.03.2021
(51) Int. Cl.: A24F 40/05, A24F 40/485, A61M 15/06, A24F 40/50, A24F 40/10

(54) **AEROSOL-GENERATING DEVICE WITH FEEDBACK CONTROL OF TRANSDUCER**
AEROSOLERZEUGUNGSVORRICHTUNG MIT RÜCKKOPPLUNGSSTEUERUNG DES WANDLERS
DISPOSITIF DE GÉNÉRATION D'AÉROSOL AYANT UNE COMMANDE DE RÉTROACTION DE TRANSDUCTEUR

(30) Priority: 05.03.2020 EP 20161319
(43) Date of publication of application: 11.01.2023
(60) Divisional application: 25164424.1
(73) Proprietor: Philip Morris Products S.A., 2000 Neuchâtel (CH)
(72) Inventor: DITTMANN, Leander, 2000 Neuchâtel (CH); ROBERT, Jacques, 2000 Neuchâtel (CH)
(74) Representative: Reddie & Grose LLP
(86) International application number: PCT/EP2021/055214
(87) International publication number: WO 2021/175869

(56) References cited:
- EP-A1- 2 883 564
- EP-A1- 2 992 968
- WO-A1-2013/173495
- WO-A1-2017/167521
- WO-A1-2022/118179
- DE-T2- 60 012 306
- GB-A- 2 542 007
- US-A1- 2016 366 946
- US-A1- 2019 014 819

## Description

The present disclosure relates to aerosol-generating devices that use a liquid aerosol-forming substrate. In particular, the disclosure relates to aerosol generating devices that use a vibrating transducer to move or atomise liquid aerosol-forming substrate.

One example of an aerosol generating device is an e-cigarette. Typically, in an e-cigarette a liquid aerosol-forming substrate is heated to generate a vapour to generate an aerosol. However, alternative designs have been proposed that use a vibrating transducer to generate droplets from a liquid aerosol-generating device, with the droplets forming an aerosol.

US 2016/0366946 A1 discloses an electronic cigarette vaporiser system that includes a piezoelectric pump with multiple piezo-actuators, in which a microcontroller independently adjusts the phase or timing or power of each voltage pulse that triggers a piezo-actuator. The microcontroller continuously or regularly monitors the efficiency or performance of the entire pump and adjusts the phase, timing, or power delivered to each piezo-actuator until or so that the optimum pumping performance is achieved.

Vibrating transducers can also be used as the basis of small liquid pumps. Small liquid pumps can be used in aerosol-generating devices, such as e-cigarettes, to deliver liquid aerosol-forming substrate from a reservoir to an atomising elements, such as a heater or vibrating transducer.

It would be desirable to optimise as far as possible the efficiency of any vibrating transducers used in an aerosol-generating device, either for generating an aerosol or for moving liquid within the device. This is particularly important in handheld aerosol-generating devices, such as e-cigarettes, which are typically battery powered, and are desirably as small as possible, but which need to generate a significant volume of aerosol on demand by a user.

It would also be desirable to be able to be able to quickly and simply detect any changing operating conditions that affect the output and efficiency of the aerosol-generating device.

According to an aspect of the present invention, there is provided an aerosol generating device. The aerosol-generating device comprises a transducer. The transducer is a piezoelectric transducer. The aerosol generating device comprises drive circuitry connected to the transducer and configured to apply an oscillating current to the transducer. The aerosol-generating device comprises control circuitry connected to the drive circuitry and configured to monitor the resonant behaviour of the transducer, the control circuitry configured to control the operation of the drive circuitry based on the resonant behaviour of the transducer.

The piezoelectric transducer forms part of a transducer assembly in a liquid pump. The transducer assembly comprises a membrane or surface configured to contact a liquid aerosol-forming substrate, the transducer assembly configured to drive the membrane or surface into vibration, the vibration of the membrane or surface forcing the liquid through an adjacent liquid valve in the liquid pump.

As used herein "resonant behaviour" means any measurable aspect of the response of the piezoelectric transducer to oscillating input signals. For example, resonant behaviour may be a resonant frequency or resonant frequencies, or a change in resonant frequency or resonant frequencies, a maximum amplitude of response or a minimum amplitude of response, a maximum or minimum input impedance, a phase response or a change in phase response.

The resonant behaviour of the transducer may be represented by one or more measureable parameters, such as input impedance or one or more frequencies of maximum amplitude response

The transducer assembly may be configured to interact with liquid aerosol-forming substrate within the device. The transducer assembly may be configured to generate an aerosol from the liquid aerosol-forming substrate. The transducer assembly may be configured to move the liquid aerosol-forming substrate.

In some embodiments, the transducer assembly comprises a perforated membrane or mesh. The transducer may be configured to drive the perforated membrane or mesh into vibration at one or more frequencies. The vibration of the perforated membrane or mesh may force liquid aerosol-forming substrate through the perforated membrane or mesh, which may result in the formation of an aerosol comprising droplets of the liquid aerosol-forming substrate.

The transducer assembly comprises a membrane or surface configured to contact a liquid aerosol-forming substrate. The transducer may be configured to drive the membrane or surface into vibration at one or more frequencies. The vibration of the membrane or surface may force the liquid through an adjacent mesh or perforated membrane, which may result in the formation of an aerosol comprising droplets of the liquid aerosol-forming substrate.

In some embodiments, the transducer assembly comprises an atomising surface configured to contact a liquid aerosol-forming substrate and electrodes on the transducer configured to generate surface acoustic waves (SAW) on the atomising surface. The SAW generate droplets of the liquid aerosol-forming substrate, which form an aerosol.

The transducer comprises a membrane or surface configured to contact a liquid aerosol-forming substrate. The transducer may be configured to drive the membrane or surface into vibration at one or more frequencies. The vibration of the membrane or surface may force the liquid through an adjacent liquid valve.

In all of these embodiments, it is beneficial to be able to control the operating frequency of the transducer or the operating power of the transducer (or both the operating frequency and the operating power of the transducer) in response to changes in the resonant behaviour of the transducer. Controlling the operating frequency in particular may be beneficial in order to improve the efficiency of the system. Controlling the operating frequency may allow generation of aerosol to be maximised.

The control circuitry may be configured to control the operation of the drive circuitry at the time that the device is first activated. The control circuitry may be configured to control the operation of the drive circuitry periodically or intermittently during operation of the device.

There are several reasons why the resonant behaviour of the piezoelectric transducer may change during operation of the device. One parameter that might affect the resonant behaviour of the transducer is temperature. The resonant frequency of the transducer assembly may change with temperature as materials of the transducer assembly expand or contract, resulting in changes in dimension and changes in residual stresses within the transducer assembly. Temperature changes of the transducer assembly may occur because of changes in ambient temperature. Temperature changes of the transducer assembly may occur because of warming of the transducer assembly as a result of energy dissipation within the device during operation. Typically the transducer assembly will warm up during operation of the device. Warming of the transducer assembly may result in a fall in the resonant frequencies of the transducer.

Other changes in ambient conditions may affect the resonant behaviour of the transducer. For example, changes in atmospheric pressure or humidity may affect the resonant behaviour of the transducer.

Changes in the material in contact with the transducer assembly may change the resonant behaviour of the transducer. In particular, changes in load on the transducer may change the resonant behaviour of the transducer. For example, changes in a volume of liquid aerosol-forming substrate in contact with the transducer assembly may change the load on the transducer. Change in the composition of the liquid aerosol-forming substrate in contact with the transducer assembly may change the load on the transducer.

The resonant behaviour of the transducer may change as a result of ageing of one or more components of the transducer assembly.

So it can be seen that changes in the resonant behaviour of the transducer assembly may be rapid or may comprise a longer term drift. It is beneficial for the device to be able to respond to both rapid changes and longer term drift.

The control circuitry may be configured to control the operation of the drive circuitry so that the oscillating current has a frequency equal to a resonant frequency of the piezoelectric transducer. Operating at a resonant frequency may allow for a maximum amount of power to be transferred to the transducer. Operating at a resonant frequency may result in a maximum amplitude of vibration and a maximum vibration velocity. This may be beneficial for generating an aerosol with desirable properties.

The control circuitry may be configured to control the operation of the drive circuitry so that the oscillating current has a frequency offset from a resonant frequency of the transducer. This may be advantageous in some circumstances. For example when the impedance of the transducer at its resonance frequency is not matched with the output impedance of the drive circuitry, then a small offset of frequency is useful to operate the system at the impedance matching point where the highest power is delivered to the transducer. This frequency may be somewhere between the resonance and anti-resonance frequencies. In this region impedance changes significantly with frequency, allowing for precise tuning.

The control circuitry may be configured to monitor the resonant behaviour of the transducer at a plurality of resonant frequencies of the transducer corresponding to different modes of vibration. The transducer may be driven at a plurality of different frequencies in order to generate aerosol droplets with different properties.

The control circuitry may be configured to monitor the resonant behaviour of the piezoelectric transducer by measuring a delivered power to the transducer or an input impedance of the transducer. At a resonant frequency, delivered power is maximised and input impedance is minimised.

The control circuitry may be configured to monitor the resonant behaviour of the piezoelectric transducer by determining zero crossing points of an output signal from the transducer or points of inflection of an output signal from the transducer. The zero crossing points or points of inflection may be used to determine a frequency of operation.

The control circuitry may comprise a phase locked loop (PLL). The phase locked loop may comprise a phase comparator and may determine a phase shift in a response from the transducer. Use of a phase locked loop may be advantageous as it does not require a microprocessor. It may be a low cost and high reliability solution.

In some embodiments, the drive and control circuitry is configured to:
a) apply a current having a drive frequency to the transducer;
b) during periodically spaced time slots, apply a current having a second frequency, which is a higher or lower frequency than the drive frequency;
c) determine if the power delivered to the transducer is increased at the second frequency when compared to the first frequency; and
d) if the power delivered is increased at the second frequency, use the second frequency as the drive frequency, otherwise retain the existing drive frequency; and
e) repeat steps a) to d).

The drive circuitry may use a second frequency that is higher than the drive frequency in alternate time slots and may use a second frequency that is lower than the drive frequency in alternate time slots.

This process results in a device that automatically tracks the resonant frequency of the transducer.

The second frequency may be higher or lower than the drive frequency by a predetermined amount.

Other options for detecting changes in resonant frequency include the use of a dedicated MEMS sensor. For example, a MEMS cantilever with low inertia can be placed in contact with a vibrating element of the transducer assembly so that the cantilever synchronizes with the oscillation of the transducer assembly and provides a corresponding electric signal. Another option is the use of real-time impedance measurement by monitoring the voltage and current in the transducer. In this case, the series and parallel modes of the transducer can be addressed individually. The transducer behaviour can be described in terms of an electric equivalent circuit consisting of a capacitance C1, an inductance L1, and a resistance R1 in series for the mechanical part and another capacitance C0 and resistance R0 for the transducer electrical part in parallel. Depending on the frequency, this circuit can run in a state where the series branch (C1,L1,R1) resonates itself (series resonance mode) or where this LCR-series resonates with the parallel C0 (parallel resonance mode). The series resonance frequency is close to the resonant frequency of the transducer and the parallel resonance frequency is close to the anti-resonance frequency of the transducer. The series mode resonance provides low impedance and results in higher current and lower voltage for the same power. Mechanically this provides large displacement amplitudes. The parallel mode resonance provides high impedance and results in lower current and higher voltage for the same power. Mechanically the losses are lower. In this case, a tuning series inductance can be added and the electro-acoustic energy transfer efficiency can be higher at anti-resonance.

The device may comprise a means to tune the resonant frequency of the transducer. For example, a membrane coupled to the piezoelectric transducer may be pre-stressed by the application of a DC bias voltage, which will alter its resonant behaviour. As components of the device age it may be beneficial to tune the resonant response of the device to the match a particular desired frequency or frequencies associated with the aerosol-forming substrate.

The control circuitry may comprise a microprocessor. The control circuitry may comprise a field programmable gate array (FPGA). The drive circuitry and the control circuitry may be integrated into a single circuit.

The control circuitry may be configured to control a carrier frequency, duty cycle, power, modulation frequency or amplitude of the oscillating current from the drive circuitry.

As described, the resonant behaviour of the transducer may be affected by the amount of liquid in contact with portion of the transducer assembly. The control circuitry may be configured to detect a reduction in the amount of liquid in contact with transducer assembly based on changes in the resonant behaviour of the transducer. This may be based on sudden changes in resonant frequency greater than a threshold amount. The control circuitry may be configured to stop operation of the drive circuitry in response to detection of a significant reduction on liquid delivered to the transducer assembly. The control circuitry may be configured to stop or modify operation of the drive circuitry based on any malfunction of the device, determined based on the resonant behaviour of the transducer.

The piezoelectric transducer may comprise a monocrystalline material. The piezoelectric transducer may comprise quartz. The piezoelectric transducer may comprise a ceramic. The ceramic may comprise barium titanate (BaTiO3). The ceramic may comprise lead zirconate titanate (PZT). The ceramic may include doping materials such as Ni, Bi, La, Nd or Nb ions. The piezoelectric transducer may be polarised. The piezoelectric transducer may be unpolarised. The piezoelectric transducer may comprise both polarised and unpolarised piezoelectric materials.

The drive circuitry may be configured to apply an oscillating current having a frequency between about 20 kHz and about 1500 kHz, or between about 50 kHz and about 1000 kHz, or between about 100 kHz and about 500 kHz. This may provide a desired aerosol-output rate and a desired droplet size.

The aerosol-generating device may be configured to generate an aerosol for user inhalation. The aerosol-generating device may be an electrically operated smoking device.

The aerosol generating device may comprise a liquid reservoir containing a liquid aerosol-forming substrate. In use, the piezoelectric transducer may be in contact with liquid from the liquid reservoir.

The aerosol-generating device may comprise a liquid storage portion containing the liquid aerosol-forming substrate reservoir. The liquid storage portion may form part of a cartridge that is separable from the remainder of the device. The liquid storage portion of the aerosol-generating system may comprise a housing that is substantially cylindrical, wherein an opening is at one end of the cylinder. The housing of the liquid storage portion may have a substantially circular cross section. The housing may be a rigid housing. As used herein, the term 'rigid housing' is used to mean a housing that is self-supporting. The rigid housing of the liquid-storage portion may provide mechanical support to the heating means.

The liquid storage portion may further comprise a carrier material within the housing for holding the aerosol-forming substrate.

The liquid aerosol-forming substrate may be adsorbed or otherwise loaded onto a carrier or support. The carrier material may be made from any suitable absorbent plug or body, for example, a foamed metal or plastics material, polypropylene, terylene, nylon fibres or ceramic. The liquid aerosol-forming substrate may be retained in the carrier material prior to use of the aerosol-generating system. The liquid aerosol-forming substrate may be released into the carrier material during use. The liquid aerosol-forming substrate may be released into the carrier material immediately prior to use.

In one example, the liquid aerosol-forming substrate is held in capillary material. A capillary material is a material that actively conveys liquid from one end of the material to another. The capillary material may be advantageously oriented in the housing to convey liquid aerosol-forming substrate to the transducer assembly. The capillary material may have a fibrous structure. The capillary material may have a spongy structure. The capillary material may comprise a bundle of capillaries. The capillary material may comprise a plurality of fibres. The capillary material may comprise a plurality of threads. The capillary material may comprise fine bore tubes. The capillary material may comprise a combination of fibres, threads and fine-bore tubes. The fibres, threads and fine-bore tubes may be generally aligned to convey liquid to the vibratable element. The capillary material may comprise sponge-like material. The capillary material may comprise foam-like material. The structure of the capillary material may form a plurality of small bores or tubes, through which the liquid can be transported by capillary action.

The capillary material may comprise any suitable material or combination of materials. Examples of suitable materials are a sponge or foam material, ceramic- or graphite-based materials in the form of fibres or sintered powders, foamed metal or plastics materials, a fibrous material, for example made of spun or extruded fibres, such as cellulose acetate, polyester, or bonded polyolefin, polyethylene, terylene or polypropylene fibres, nylon fibres or ceramic. The capillary material may have any suitable capillarity and porosity so as to be used with different liquid physical properties. The liquid aerosol-forming substrate has physical properties, including but not limited to viscosity, surface tension, density, thermal conductivity, boiling point and atom pressure, which allow the liquid to be transported through the capillary material by capillary action. The capillary material may be configured to convey the aerosol-forming substrate to the transducer assembly.

The carrier material may abut the transducer assembly. The liquid aerosol-forming substrate may be transported by capillary action from the liquid storage portion to the transducer assembly.

Alternatively, or in addition, the device may comprise a pump. Liquid aerosol-forming substrate may be delivered to the transducer assembly from the reservoir by the pump.

The aerosol-generating device may comprise liquid aerosol-forming substrate in the housing of the liquid-storage portion. The liquid aerosol-forming substrate is a substrate capable of releasing volatile compounds that can form an aerosol. The volatile compounds may be released by moving the liquid aerosol-forming substrate through the passages of the vibratable element.

The liquid aerosol-forming substrate may comprise nicotine. The nicotine containing liquid aerosol-forming substrate may be a nicotine salt matrix. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the aerosol-forming substrate upon heating. The liquid aerosol-forming substrate may comprise homogenised tobacco material. The liquid aerosol-forming substrate may comprise a non-tobacco-containing material. The liquid aerosol-forming substrate may comprise homogenised plant-based material.

The liquid aerosol-forming substrate may comprise at least one aerosol-former. An aerosol-former is any suitable known compound or mixture of compounds that, in use, facilitates formation of a dense and stable aerosol and that is substantially resistant to thermal degradation at the temperature of operation of the system. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Aerosol formers may be polyhydric alcohols or mixtures thereof, such as triethylene glycol, 1,3-butanediol and glycerine. The liquid aerosol-forming substrate may comprise other additives and ingredients, such as flavourants.

The aerosol-forming substrate may comprise nicotine and at least one aerosol former. The aerosol former may be glycerine. The aerosol-former may be propylene glycol. The aerosol former may comprise both glycerine and propylene glycol. The aerosol-forming substrate may have a nicotine concentration of between about 2% and about 10%.

The aerosol-forming substrate may have a dynamic viscosity (µ) at a temperature of 20°C of between about 0.4 mPa.S (0.4 mPl, 0.4 cP) and about 1000 mPa.S (1000 mPl, 1000 cP), or between about 1 mPa.S and about 100 mPa.S, or about 1.5 mPa.S and about 10 mPa.S.

The aerosol-generating device may comprise a power supply. The power supply may be a battery. The battery may be a Lithium based battery, for example a Lithium-Cobalt, a Lithium-Iron-Phosphate, a Lithium Titanate or a Lithium-Polymer battery. The battery may be a Nickel-metal hydride battery or a Nickel cadmium battery. The power supply may be another form of charge storage device such as a capacitor. The power supply may require recharging and be configured for many cycles of charge and discharge. The power supply may have a capacity that allows for the storage of enough energy for one or more smoking experiences; for example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the heating means and actuator.

The aerosol-generating device may be portable. The aerosol-generating device may have a size comparable to a conventional cigar or cigarette. The aerosol-generating system may have a total length between about 30 mm and about 150 mm. The aerosol-generating device may have an external diameter between about 5 mm and about 30mm.

The aerosol-generating device may comprise a housing. The housing may be elongate. The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. The material may be light and non-brittle.

The housing may comprise a cavity for receiving the power supply. The housing may comprise a mouthpiece. The mouthpiece may comprise at least one air inlet and at least one air outlet. The mouthpiece may comprise more than one air inlet.

The action of the transducer assembly on the liquid aerosol-forming substrate may heat the aerosol-forming substrate. This may be desirable when it is desirable to deliver a warm aerosol to a user. Alternatively, or in addition, the device may include a heater. The heater may heat the liquid aerosol-forming substrate before it reaches the transducer assembly, at the transducer assembly or after it has formed an aerosol.

In another aspect of the invention, there is provided a method of operating an aerosol generating device. The device comprises a piezoelectric transducer. The device comprises drive circuitry connected to the piezoelectric transducer. The device comprises control circuitry configured to monitor a parameter of the piezoelectric transducer and connected to the drive circuitry. The method comprises applying an oscillating current to the transducer using the drive circuity. The method further comprises monitoring the resonant behaviour of the piezoelectric transducer using the control circuitry. The method further comprises controlling the operation of the drive circuitry based on the monitored resonant behaviour of the piezoelectric transducer.

The piezoelectric transducer is part of a transducer assembly. The transducer assembly is in a liquid pump. The transducer assembly comprises a membrane or surface configured to contact a liquid aerosol-forming substrate. The piezoelectric transducer is configured to drive the membrane or surface into vibration. The vibration of the membrane or surface forces the liquid through an adjacent liquid valve in the liquid pump.

The method may comprise stopping the operation of the drive circuitry based on the monitored resonant behaviour of the piezoelectric transducer. The method may comprise controlling a carrier frequency, duty cycle, power, modulation frequency or amplitude of the oscillating current from the drive circuitry.

The step of monitoring the resonant behaviour may comprise periodically applying an oscillating current with a different frequency and determining the resonant behaviour of the transducer at the different frequency. The method may comprise applying an oscillating current comprising a plurality of sinusoidal frequencies.

The invention may provide the advantage of efficient operation throughout operation, regardless of changing loads on the transducer and changing ambient conditions or device conditions. The invention may also provide a means for detecting malfunctions and abnormal operating conditions, such as a reduced supply of liquid aerosol-forming substrate.

Examples will now be further described with reference to the figures in which:
Figure 1 illustrates a feedback control system in accordance with the invention;
Figure 2 is a schematic illustration of an aerosol generating device in accordance with the invention;
Figure 3 illustrates a transducer assembly for use in the system of Figure 2;
Figure 4 is a schematic plot showing a change in response of the transducer over time;
Figure 5 illustrates an example of a drive and control circuit implementing feedback control; and
Figure 6 is a schematic illustration of a an aerosol-generating device comprising a piezoelectric pump in accordance with the invention.

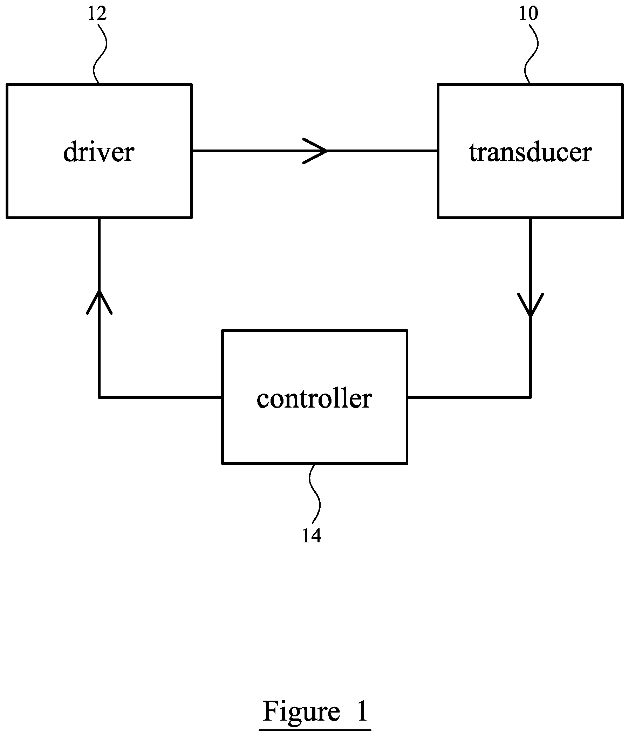

Figure 1 is a schematic illustration of a feedback control loop in accordance with the invention. The feedback loop comprises a transducer 12, driver circuitry 14 and control circuitry 14. The transducer in this example is a piezoelectric transducer. The transducer is coupled to and vibrates a membrane for generating an aerosol from a liquid supply. The transducer 12 is driven at a particular drive frequency by the drive circuitry 12. The drive circuitry 12 supplies an oscillating current to the transducer, which causes it to expand and contract. This in turn causes the membrane to vibrate.

The transducer has one or more resonant frequencies. The resonant frequencies depend on several factors including the load on the transducer. The load on the transducer depends on the properties of the membrane and any load on the membrane. The resonant frequencies also depend on temperature, for example.

In order to ensure that the transducer is being driven at a resonant frequency by the drive circuitry, the control circuitry 14 completes a feedback loop. The control circuitry receives a feedback parameter from the transducer, for example a phase shift or a vibration amplitude. The value of the feedback parameter varies depending on how close the drive frequency is to the resonant frequency of the transducer. The drive circuitry 12 adjusts the drive frequency of the oscillating current applied to the transducer 10 and the effect of that change in drive frequency on the feedback parameter is monitored by the control circuitry. The control circuitry then sends a control signal to the drive circuitry and the drive circuitry adjusts the frequency of the applied oscillating current based on the control signal, in order to achieve particular effect. In many cases it is desirable to drive the transducer as close to a resonant frequency as possible. But in some circumstances it may be desirable to drive the transducer at a particular offset from a resonant frequency or at a frequency between resonant and anti-resonant frequencies. The control circuitry may comprise filters, a microcontroller or any analog or digital means to process the feedback parameter in order generate the control signal.

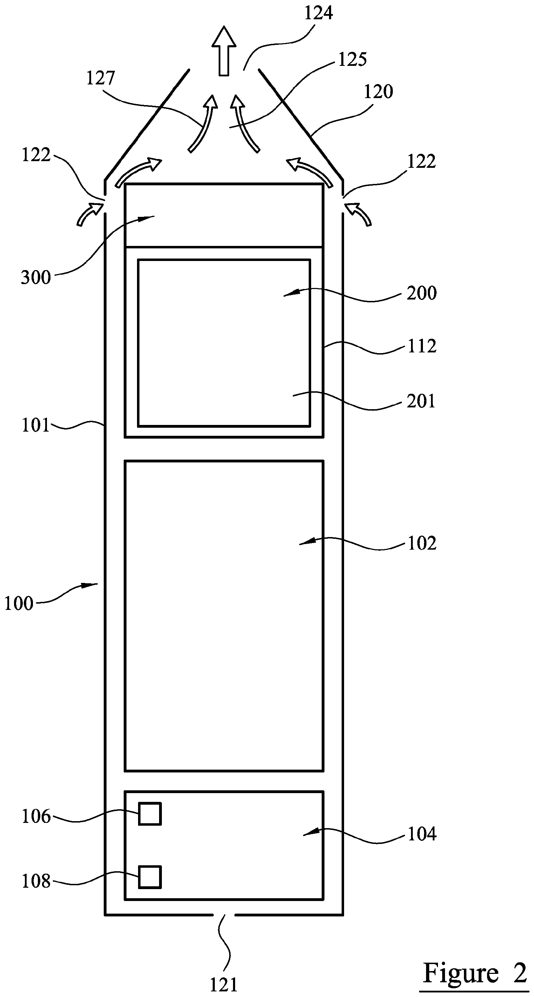

Figure 2 is a schematic view of a first embodiment of an aerosol-generating device according to the invention, that incorporates the feedback control illustrated in Figure 1. Figure 2 is schematic in nature. In particular, the components shown are not necessarily to scale either individually or relative to one another. The aerosol-generating device comprises a reusable device portion 100 in cooperation with a cartridge 200, which is preferably disposable. In Figure 2, the device is an electrically operated smoking system.

The device portion 100 comprises a main body having a housing 101. The housing 101 is substantially circularly cylindrical and has a longitudinal length of about 100 mm and an external diameter of about 20 mm, comparable to a conventional cigar. In the device, there is provided an electric power supply in the form of battery 102 and electric control circuitry 104. The electric control circuitry 104 includes the drive circuitry and control circuitry for the transducer, as described with reference to Figure 1. The main body housing 101 also defines a cavity 112 into which the cartridge 200 is received.

The cartridge 200 (shown in schematic form in Figure 2) comprises a rigid housing defining a liquid storage portion 201. The liquid storage portion 201 holds a liquid aerosol-forming substrate (not shown). The housing of the cartridge 200 is fluid impermeable but has an open end (not shown) that is coverable by a removable lid (not shown) when the cartridge is removed from the device 100. The lid may be removed from the cartridge 200 before insertion of the cartridge into the device. The cartridge 200 includes keying features (not shown) to ensure the cartridge 200 cannot be inserted into the device upside-down.

The device portion 100 also includes a mouthpiece portion 120. The mouthpiece portion 120 is connected to the main body housing 101 by a hinged connection in this example, but any kind of connection may be used, such as a snap fitting or a screw fitting. The mouthpiece portion 120 comprises a plurality of air inlets 122, an air outlet 124 and an aerosol forming chamber 125, and an atomiser 300 mounted therein (shown schematically in Figure 2). Air inlets 122 are defined between the mouthpiece portion 120 and the main body housing 101 of the device 100 when the mouthpiece portion is in a closed position, as shown in Figure 2. An air-flow route 127 is formed from the air inlets 122 to the air outlet 124 via the aerosol forming chamber 125 and the atomiser 300, as shown in Figure 2 by the arrows.

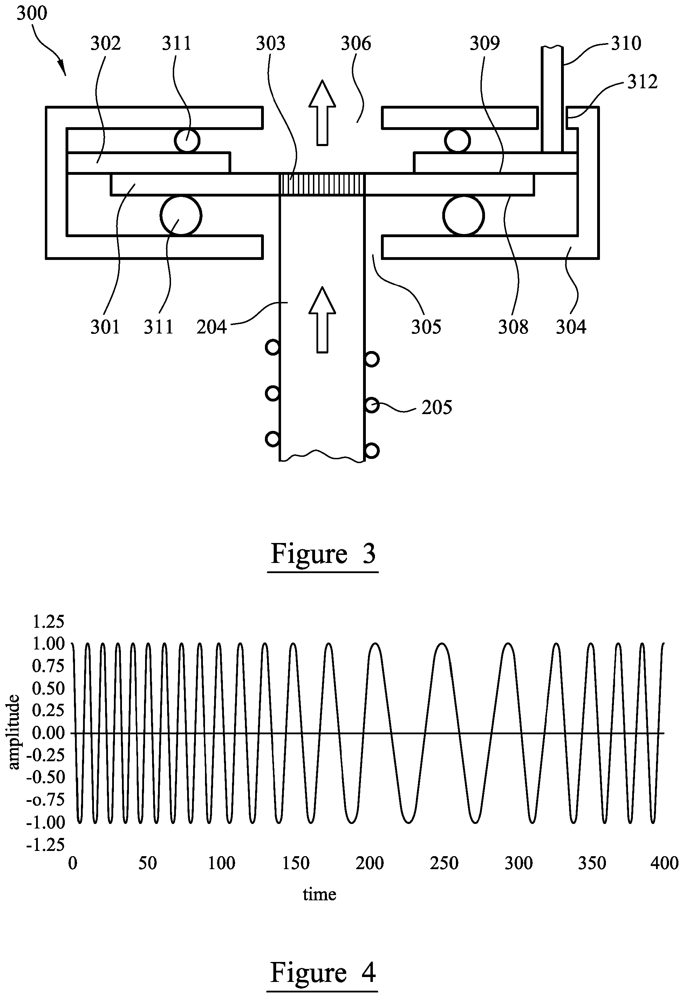

As shown in Figure 3, the atomiser 300 comprises a vibratable element 301 and transducer 302 housed inside an atomiser housing 304. Atomiser housing 304 comprises a hollow cylindrical box, having an inlet opening 305 and an outlet opening 306 arranged in coaxial alignment at opposite sides of the housing 304. The housing 304 is removably connected to the mouthpiece 120 of the device portion 100 by a screw thread connection (not shown). A male screw thread (not shown) is provided at an outer surface of the atomiser housing 304, that is complimentary to a female screw thread (not shown) on an inner surface of the mouthpiece 120. Atomiser 300 is removable from the mouthpiece portion 120 of the device portion for disposal or for cleaning.

Vibratable element 301 comprises a substantially circular aluminium disc, having a thickness of about 2 mm and a diameter of about 15 mm.

A plurality of passages 303 extends from an inlet side 308 to an opposing outlet side 309 of the vibratable element. The plurality of passages form an array having a substantially circular shape. The substantially circular array has a diameter of about 7 mm, and is arranged substantially centrally in the element 301.

The passages (not shown) have a substantially circular cross-section and are tapered from the inlet side 308 to the outlet side 309 of the vibratable element 301. The passages have a diameter at the inlet side of about 8 µm and a diameter at the outlet side of about 6 µm. The passages are typically formed by high-speed laser drilling. The plurality of passages is comprised of about 4000 passages arranged with equal spacing across the array.

Transducer 302 comprises a piezoelectric transducer. The piezoelectric transducer is a substantially circular annular disc of piezoelectric material, typically zirconate titanate (PZT). The piezoelectric transducer has a thickness of about 2 mm, an outer diameter of about 17 mm and an inner diameter of about 8 mm.

As shown in Figure 3, the transducer 302 is in direct contact with the vibratable element 301, at the outlet side 309 of the vibratable element. The inner diameter of the piezoelectric transducer 302 circumscribes the array of passages 303 of the vibratable element 301, such that the open ends of the passages at the outlet side are not covered by the piezoelectric transducer 302. In other embodiments (not shown) it is envisaged that the piezoelectric transducer 302 may be in direct contact with the vibratable element 301 at the inlet side 308.

The vibratable element 301 and piezoelectric transducer 302 are supported within the atomiser housing 304 by a pair of elastomeric O-rings 311, which allow the vibratable element 301 and the piezoelectric transducer 302 to vibrate within the housing 304. The vibratable element 301 and piezoelectric transducer 302 are held together by pressure from the opposing O-rings 311. However, in other embodiments (not shown) the vibratable element 301 and the piezoelectric transducer 302 may be bonded by any suitable means, such as an adhesive layer.

The vibratable element 301 and the piezoelectric transducer 302 are arranged within the atomiser housing 304 such that the array of passages 303 is in coaxial alignment with the inlet and outlet openings 305, 306 of the housing 304.

One or more spring pins 310 extend through an opening 312 in the atomiser housing 304 to provide electrical connection of the piezoelectric transducer 302 to the control circuitry 104 and the battery 102 of the device 100. The one or more spring pins 310 are held in contact with the piezoelectric transducer 302 by pressure, rather than by a mechanical connection so that good electrical contact is maintained during vibration of the piezoelectric transducer 302.

In use, when the atomiser 300 is removably connected to the mouthpiece portion 120 of the device portion 100 and the cartridge 200 is received in the cavity 112 of the device, an elongate capillary body (not shown in Figure 2) extends from the liquid storage portion 201 of the cartridge 200 to the atomiser 300 to fluidly connect the cartridge 200 to the atomiser 300. As shown in Figure 3, the elongate capillary body 204 extends into the atomiser housing 304 and abuts the inlet side 308 of the vibratable element 301 at the array of passages 303. Heating means is provided in the liquid storage portion in the form of a coil heater 205 surrounding the capillary body 204. Note that the coil heater is only shown schematically in Figure 3. The coil heater 205 is connected to the electric circuitry 104 and battery 102 of the device 100 via connections (not shown), which may pass along the outside of the liquid storage portion 200, although this is not shown in Figure 2 or Figure 3.

In use, liquid aerosol-forming substrate (not shown) is conveyed by capillary action from the liquid storage portion 201 from the end of the capillary body 204 which extends into the liquid storage portion 201, past the heater coil 205, and to the other end of the capillary body 204, which extends into the atomiser housing 304 and abuts the vibratable element 301 at the inlet side 308 at the array of passages 303.

When a user draws on the air outlet 124 of the mouthpiece portion 120, ambient air is drawn through air inlets 122. In the embodiment of Figure 2, a puff detection device 106 in the form of a microphone, is also provided as part of the control electronics 104. A small air flow is drawn through a sensor inlet 121 in the main body housing 101, past the microphone 106 and up into the mouthpiece portion 120. When a puff is detected by the electric circuitry 104, the electric circuitry 104 activates the heater coil 205 and the piezoelectric transducer 302. The battery 102 supplies electrical energy to the coil heater 205 to heat the capillary body 204 surrounded by the coil heater.

The battery 102 further supplies electrical energy to the piezoelectric transducer 302, under the control of the drive and control circuitry, which vibrates, deforming in the thickness direction. The piezoelectric transducer 302 typically vibrates at around approximately 150 kHz. The drive current supplied to the transducer has an initial frequency and wave shape based on parameters stored in memory. During manufacture of the device the frequency response of the transducer assembly, including the vibratable element 301, can be characterised and an initial frequency and waveform set. The piezoelectric transducer 302 transmits the vibrations to the vibratable element 301, which vibrates, also deforming in the thickness direction. An LED 108 is also activated to indicate that the device is activated. As will be described, during operation the feedback control loop is used to adjust the drive current supplied to the transducer in response to detected changes in resonant behaviour.

The coil heater 205 heats the liquid aerosol-forming substrate being conveyed along the capillary body, past the coil heater 205, to a predetermined temperature of about 45°C.

The vibrations in the vibratable element deform the plurality of passages 303, which draws heated liquid aerosol-forming substrate from the capillary body 204, through the plurality of passages 303 at the inlet side 308 of the vibratable element 301, and ejects atomised droplets of liquid aerosol-forming substrate from the passages at the outlet side 309 of the vibratable element 301, forming an aerosol. At the same time, the heated liquid being atomised is replaced by further liquid moving along the capillary body 204 by capillary action. (This is sometimes referred to as 'pumping action'). The aerosol droplets ejected from the vibratable element 301 mix with and are carried in the air flow 127 from the inlets 122 in the aerosol forming chamber 125, and are carried towards the air outlet 124 of the mouthpiece 120 for inhalation by the user.

As previously described, during operation the resonant response of the transducer may change. Figure 4 is a schematic plot of a sensed parameter from the transducer, showing a change in frequency over time. The distance in time between successive crossings of the signal through zero is a measure of frequency and may be used to synchronize the driving signal with the working frequency of the transducer, in this example its resonant frequency. The signal may be, for example, the current measured by a sense resistor in series with the transducer. In that case the amplitude may have a unit of Amperes for the current or the amplitude may be normalized, for example by its maximum value, in which case the unit of the amplitude is 1. Time may have, for example, the unit of milliseconds or microseconds depending on the characteristic frequency working range covered by the transducer.

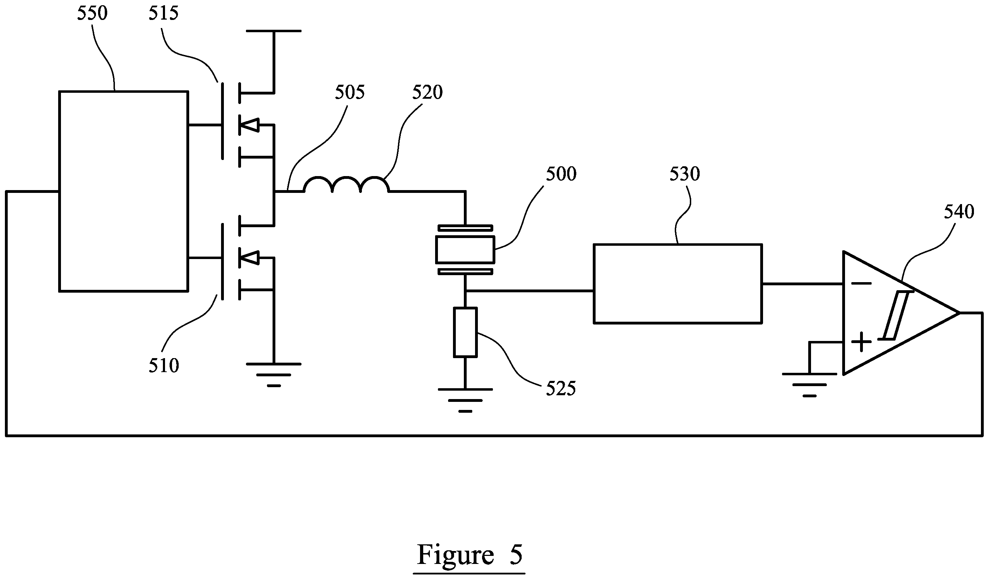

One specific example of a possible implementation of such a feedback loop is shown in Fig. 5. The transducer 500, which is connected to the vibratable perforated plate in the embodiment of Figure 3, is driven by a half-bridge 505, consisting of two power MOSFETs 510, 515. An optional series inductor 520, for example a 10 microhenrys inductor, may be used between the half-bridge and the transducer to tune impedance. The current sense resistor 525, of for example 1 Ohm, may be placed at the low end of the transducer 500. The voltage measured across the current sense resistor is proportional to the current through the transducer. This voltage signal may be filtered and amplified by filter and gain stage 530. The filter and gain stage 530 may comprise, for example, a low pass filter, in order to cut off high frequency harmonics, and an FET amplifier, for example AD823, to amplify the signal. A comparator 540 creates the feedback signal, in this example a square wave signal as an appropriate input waveform for the gate driver 550. The gate driver 550 may, for example, be an IC of type LT1162, and drives the half-bridge 505. As the change in frequency is detected and sent back to the driver, the transducer 500 will be driven always at its working frequency, for example its resonant frequency. The drive and control circuitry shown in Figure 5 can be incorporated into the control circuitry 104 shown in Figure 2.

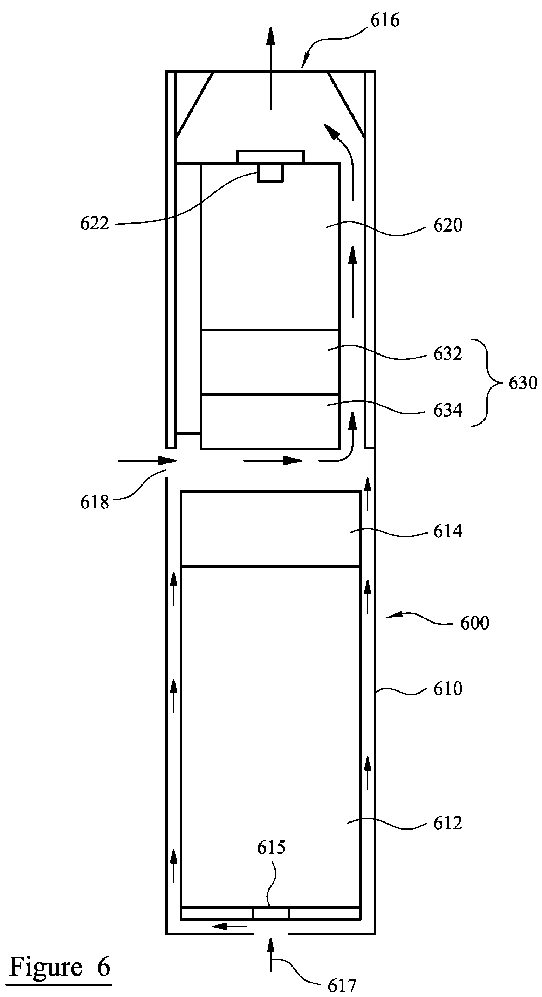

Figure 6 is an illustration of an aerosol-generating device according to another embodiment of the present invention. Figure 6 is schematic in nature. In particular, the components shown are not necessarily to scale either individually or relative to one another. The device of Figure 6 generates aerosol by heating a liquid aerosol-forming substrate using a heater. However the device includes a pump that uses a piezoelectric transducer to transport the liquid aerosol-forming substrate to the heater.

The device is a handheld, electrically operated smoking device 600 and comprises a housing 610. Within the housing 610 there is an electric power supply in the form of battery 612 and control circuitry 614. Also within the housing there is a liquid reservoir 620 containing a liquid aerosol-forming substrate that is vapourised in order to form an aerosol that is inhaled by a user. An atomiser assembly 630 is provided within the housing, coupled to the liquid reservoir 620. The atomiser assembly comprises a vapouriser 634, in this example an electrical heater, and a pump 632 positioned to pump liquid from the liquid reservoir 620 to the vapouriser 634. Both the pump 632 and the electric heater 634 are provided with power from the battery 612 under the control of the control circuitry 614, as will be described.

The housing 610 includes an air inlet 618 and an air outlet 616. The air outlet 616 is provided at a mouthpiece end of the housing. In use, a user sucks on the mouthpiece end of the housing. This draws air through the air inlet 618 into the housing, past the vapouriser 634 and out through the outlet 616 into the user's mouth. The air drawn past the vapouriser entrains vapourised aerosol-forming substrate. The vapourised aerosol-forming substrate cools to form an aerosol as it moves through the device and into the user's mouth.

Activation of the heater may be controlled directly by a user pressing a button on the housing 610. Alternatively, the system may comprise an airflow sensor, such as a microphone 615, that detects airflow through the system and the heater may be activated based on signals from the airflow sensor. When a user draws air through the system, herein referred to as puffing, air flows past the air flow sensor 615. If the airflow detected by the airflow sensor exceeds a threshold value, then the control circuitry may activate the heater by supplying power to the heater. The control circuitry may supply power to the heater for a predetermined time period or may supply power to the heater for as long as the detected airflow exceeds a threshold. The control circuitry may include temperature sensing means, such as a dedicated temperature sensor or by monitoring an electrical resistance of the heater. The control circuitry may then supply power to the heater to raise the temperature of the heater to within a desired temperature range. The temperature should be sufficient to vapourise the aerosol-forming substrate but not so high that there is a significant risk of combustion.

The liquid in this example comprises a mixture of water, glycerol, propylene glycol, nicotine and flavourings. The liquid is held within the liquid reservoir 620. The liquid reservoir is provided as a cartridge that can be replaced when the liquid has been used up. In order to prevent leakage of the liquid, both before and during use, the liquid reservoir has a housing formed from a rigid plastics material, and is liquid tight. As used herein "rigid" means that the housing that is self-supporting. In this example, the reservoir is formed by 3D printing using an acrylic based photopolymer. The cartridge needs to be robust and able to withstand significant loads during shipping and storage. However, because the liquid reservoir housing is sealed and rigid, the liquid reservoir has a fixed internal volume. A reduction in the internal pressure inside the liquid reservoir as liquid is removed by the pump, could detrimentally affect the ability to pump liquid out of the reservoir. In order to prevent a significant drop in pressure, the liquid reservoir has an equalising air inlet valve 622. The equalising valve 622 allows air into the liquid reservoir when the pressure difference between the inside the reservoir and outside of the reservoir exceeds a threshold pressure difference.

The pump may be activated in the same way as the heater. For example the control circuitry may supply power to the pump for the same time periods as power is supplied to the heater. Alternatively, the control circuitry may supply power to the pump in periods immediately following activation of the heater.

The control circuitry 614 includes a feedback loop as illustrated in Figure 1 for control of the pump 632. The pump 632 includes a piezoelectric transducer that drives a flexible diaphragm to vibrate. Vibration of the flexible diaphragm pushes liquid aerosol-forming substrate out of a pump chamber through an outlet valve as it reduces the chamber volume, and draws liquid aerosol-forming substrate into the pump chamber through an inlet valve as it increase chamber volume. In order maximise pumping efficiency, it is advantageous to operate the pump 632 at or close to a resonant frequency of the transducer. However, as previously described, the resonant frequency of the transducer may change for a number of reasons.

Changes in the resonant frequency of the transducer due to temperature changes, other environmental changes or aging can be monitored and the drive signal modified accordingly using one of the feedback mechanisms described above.

Change to the resonant frequency of the transducer due to insufficient liquid being drawn into the pump chamber can be detected as a sudden change in resonant behaviour, such as a change greater than a predetermined threshold between two measurement cycles. If a sudden change in resonant behaviour is detected, operation of the pump and the heater can be stopped until a new liquid reservoir is placed in the device.

## Claims

1. An aerosol generating device comprising:
a piezoelectric transducer (10);
drive circuitry (12) connected to the piezoelectric transducer (10) and configured to apply an oscillating current to the transducer (10); and
control circuitry (14) connected to the drive circuitry (12) and configured to monitor the resonant behaviour of the piezoelectric transducer (10), the control circuitry (14) configured to control the operation of the drive circuitry based on the resonant behaviour of the piezoelectric transducer; wherein
the piezoelectric transducer (10) forms part of a transducer assembly in a liquid pump, and the transducer assembly comprises a membrane or surface configured to contact a liquid aerosol-forming substrate, the transducer assembly configured to drive the membrane or surface into vibration, the vibration of the membrane or surface forcing the liquid through an adjacent liquid valve in the liquid pump.

2. An aerosol generating device according to claim 1, wherein the control circuitry (14) is configured to control the operation of the drive circuitry (12) so that the oscillating current has a frequency equal to a resonant frequency of the piezoelectric transducer (10).

3. An aerosol generating device according to claim 1, wherein the control circuitry (14) is configured to control the operation of the drive circuitry (12) so that the oscillating current has a frequency offset from a resonant frequency of the piezoelectric transducer (10).

4. An aerosol generating device according to any one of the preceding claims, wherein the control circuitry (14) is configured to monitor the resonant behaviour of the piezoelectric transducer (10) at a plurality of resonant frequencies of the piezoelectric transducer corresponding to different modes of vibration.

5. An aerosol generating device according to any one of the preceding claims, wherein the control circuitry (14) is configured to monitor the resonant behaviour of the piezoelectric transducer (10) by measuring a delivered power to the piezoelectric transducer (10) or an impedance of the piezoelectric transducer.

6. An aerosol generating device according to any one of the preceding claims, wherein the drive circuitry (12) and control circuitry (14) comprises a phase locked loop (PLL).

7. An aerosol generating device according to any preceding claim, wherein the piezoelectric transducer (10) is an aerosol generating element configured to generate an aerosol from a liquid aerosol-forming substrate.

8. An aerosol generating device according to claim 7, wherein the piezoelectric transducer (10) comprises a perforated plate.

9. An aerosol generating device according to any one of the preceding claims, comprising a liquid reservoir (620) containing a liquid aerosol-forming substrate, wherein, in use, the piezoelectric transducer is in contact with liquid from the liquid reservoir.

10. An aerosol generating device according to claim 9, wherein the liquid comprises a mixture of different compounds.

11. An aerosol generating device according to claim 9 or 10, wherein the control circuitry (14) is configured to detect reduction in amount of liquid in contact with piezoelectric transducer based on changes in the resonant behaviour of the piezoelectric transducer.

12. An aerosol generating device according to any one of the preceding claims, wherein the aerosol generating device is an e-cigarette.

13. An aerosol generating device according to any one of the preceding claims, wherein the oscillating current comprises a first frequency modulated with at least one other frequency.

14. A method of operating an aerosol generating device, the device comprising a transducer assembly in a liquid pump, wherein the transducer assembly comprises a piezoelectric transducer (10), a membrane or surface configured to contact a liquid aerosol-forming substrate, the piezoelectric transducer configured to drive the membrane or surface into vibration, the vibration of the membrane or surface forcing the liquid through an adjacent liquid valve in the liquid pump, drive circuitry (12) connected to the piezoelectric transducer and control circuitry (14) configured to monitor a parameter of the piezoelectric transducer and connected to the drive circuitry, the method comprising:
applying an oscillating current to the transducer using the drive circuity; and
monitoring the resonant behaviour of the piezoelectric transducer using the control circuitry, and
controlling the operation of the drive circuitry based on the monitored resonant behaviour of the piezoelectric transducer.

## Patentansprüche

1. Aerosolerzeugungsvorrichtung, umfassend:
einen piezoelektrischen Wandler (10);
Treiberschaltung (12), die mit dem piezoelektrischen Wandler (10) verbunden und dazu eingerichtet ist, einen Schwingstrom an den Wandler (10) anzulegen; und
Regelschaltung (14), die mit der Treiberschaltung (12) verbunden und dazu eingerichtet ist, das Resonanzverhalten des piezoelektrischen Wandlers (10) zu überwachen, die Regelschaltung (14) dazu eingerichtet ist, den Betrieb der Treiberschaltung basierend auf dem Resonanzverhalten des piezoelektrischen Wandlers zu regeln; wobei
der piezoelektrische Wandler (10) einen Teil einer Wandlerbaugruppe in einer Flüssigkeitspumpe bildet, und die Wandlerbaugruppe eine Membran oder Fläche umfasst, die dazu eingerichtet ist, ein flüssiges aerosolbildendes Substrat zu kontaktieren, die Wandlerbaugruppe dazu eingerichtet ist, die Membran oder Fläche in Schwingung zu versetzen, die Schwingung der Membran oder Fläche die Flüssigkeit durch ein angrenzendes Flüssigkeitsventil in der Flüssigkeitspumpe drückt.

2. Aerosolerzeugungsvorrichtung nach Anspruch 1, wobei die Regelschaltung (14) dazu eingerichtet ist, den Betrieb der Treiberschaltung (12) derart zu regeln, dass der Schwingstrom eine Frequenz aufweist, die gleich einer Resonanzfrequenz des piezoelektrischen Wandlers (10) ist.

3. Aerosolerzeugungsvorrichtung nach Anspruch 1, wobei die Regelschaltung (14) dazu eingerichtet ist, den Betrieb der Treiberschaltung (12) derart zu regeln, dass der Schwingstrom eine gegenüber einer Resonanzfrequenz des piezoelektrischen Wandlers (10) versetzte Frequenz aufweist.

4. Aerosolerzeugungsvorrichtung nach einem beliebigen der vorhergehenden Ansprüche, wobei die Regelschaltung (14) dazu eingerichtet ist, das Resonanzverhalten des piezoelektrischen Wandlers (10) bei einer Vielzahl von Resonanzfrequenzen des piezoelektrischen Wandlers zu überwachen, die unterschiedlichen Schwingungsmodi entsprechen.

5. Aerosolerzeugungsvorrichtung nach einem beliebigen der vorhergehenden Ansprüche, wobei die Regelschaltung (14) dazu eingerichtet ist, das Resonanzverhalten des piezoelektrischen Wandlers (10) durch Messen einer an den piezoelektrischen Wandler (10) abgegebenen Energie oder einer Impedanz des piezoelektrischen Wandlers zu überwachen.

6. Aerosolerzeugungsvorrichtung nach einem beliebigen der vorhergehenden Ansprüche, wobei die Treiberschaltung (12) und die Regelschaltung (14) eine Phasenregelschleife (PLL) umfassen.

7. Aerosolerzeugungsvorrichtung nach einem beliebigen vorhergehenden Anspruch, wobei der piezoelektrische Wandler (10) ein aerosolerzeugendes Element ist, das dazu eingerichtet ist, ein Aerosol aus einem flüssigen aerosolbildenden Substrat zu erzeugen.

8. Aerosolerzeugungsvorrichtung nach Anspruch 7, wobei der piezoelektrische Wandler (10) eine perforierte Platte umfasst.

9. Aerosolerzeugungsvorrichtung nach einem beliebigen der vorhergehenden Ansprüche, umfassend einen Flüssigkeitsvorratsbehälter (620), der ein flüssiges aerosolbildendes Substrat enthält, wobei der piezoelektrische Wandler in Gebrauch in Kontakt mit Flüssigkeit aus dem Flüssigkeitsvorratsbehälter steht.

10. Aerosolerzeugungsvorrichtung nach Anspruch 9, wobei die Flüssigkeit ein Gemisch aus verschiedenen Verbindungen umfasst.

11. Aerosolerzeugungsvorrichtung nach Anspruch 9 oder 10, wobei die Regelschaltung (14) dazu eingerichtet ist, basierend auf Änderungen in dem Resonanzverhalten des piezoelektrischen Wandlers eine Verringerung der Flüssigkeitsmenge zu detektieren, die in Kontakt mit dem piezoelektrischen Wandler steht.

12. Aerosolerzeugungsvorrichtung nach einem beliebigen der vorhergehenden Ansprüche, wobei die Aerosolerzeugungsvorrichtung eine E-Zigarette ist.

13. Aerosolerzeugungsvorrichtung nach einem beliebigen der vorhergehenden Ansprüche, wobei der Schwingstrom eine erste Frequenz umfasst, die mit wenigstens einer anderen Frequenz moduliert ist.

14. Verfahren für ein Betreiben einer Aerosolerzeugungsvorrichtung, die Vorrichtung umfassend eine Wandlerbaugruppe in einer Flüssigkeitspumpe, wobei die Wandlerbaugruppe einen piezoelektrischen Wandler (10), eine Membran oder Fläche, die dazu eingerichtet ist, ein flüssiges aerosolbildendes Substrat zu kontaktieren, der piezoelektrische Wandler dazu eingerichtet ist, die Membran oder Fläche in Schwingung zu versetzen, die Schwingung der Membran oder Fläche die Flüssigkeit durch ein angrenzendes Flüssigkeitsventil in der Flüssigkeitspumpe drückt, eine Treiberschaltung (12), die mit dem piezoelektrischen Wandler verbunden ist, und eine Regelschaltung (14) umfasst, die dazu eingerichtet ist, einen Parameter des piezoelektrischen Wandlers zu überwachen und mit der Treiberschaltung verbunden ist, das Verfahren umfassend:
Anlegen eines Schwingstroms an den Wandler unter Verwendung der Treiberschaltung; und
Überwachen des Resonanzverhaltens des piezoelektrischen Wandlers unter Verwendung der Regelschaltung, und
Regeln des Betriebs der Treiberschaltung basierend auf dem überwachten Resonanzverhalten des piezoelektrischen Wandlers.

## Revendications

1. Dispositif de génération d'aérosol comprenant :
un transducteur piézoélectrique (10) ;
une circuiterie d'attaque (12) raccordée au transducteur piézoélectrique (10) et configurée pour appliquer un courant oscillant au transducteur (10) ; et
une circuiterie de commande (14) raccordée à la circuiterie d'attaque (12) et configurée pour surveiller le comportement de résonance du transducteur piézoélectrique (10), la circuiterie de commande (14) étant configurée pour commander le fonctionnement de la circuiterie d'attaque sur la base du comportement de résonance du transducteur piézoélectrique ; dans lequel
le transducteur piézoélectrique (10) fait partie d'un ensemble transducteur dans une pompe à liquide, et l'ensemble transducteur comprend une membrane ou une surface configurée pour entrer en contact avec un substrat formant aérosol liquide, l'ensemble transducteur étant configuré pour exciter la membrane ou la surface pour la mettre en vibration, la vibration de la membrane ou de la surface forçant le liquide à passer à travers une vanne à liquide adjacente dans la pompe à liquide.

2. Dispositif de génération d'aérosol selon la revendication 1, dans lequel la circuiterie de commande (14) est configurée pour commander le fonctionnement de la circuiterie d'attaque (12) de sorte que le courant oscillant a une fréquence égale à une fréquence de résonance du transducteur piézoélectrique (10).

3. Dispositif de génération d'aérosol selon la revendication 1, dans lequel la circuiterie de commande (14) est configurée pour commander le fonctionnement de la circuiterie d'attaque (12) de sorte que le courant oscillant a une fréquence décalée par rapport à une fréquence de résonance du transducteur piézoélectrique (10).

4. Dispositif de génération d'aérosol selon l'une quelconque des revendications précédentes, dans lequel la circuiterie de commande (14) est configurée pour surveiller le comportement de résonance du transducteur piézoélectrique (10) à une pluralité de fréquences de résonance du transducteur piézoélectrique correspondant à différents modes de vibration.

5. Dispositif de génération d'aérosol selon l'une quelconque des revendications précédentes, dans lequel la circuiterie de commande (14) est configurée pour surveiller le comportement de résonance du transducteur piézoélectrique (10) en mesurant une puissance délivrée au transducteur piézoélectrique (10) ou une impédance du transducteur piézoélectrique.

6. Dispositif de génération d'aérosol selon l'une quelconque des revendications précédentes, dans lequel la circuiterie d'attaque (12) et la circuiterie de commande (14) comprennent une boucle à phase asservie (PLL).

7. Dispositif de génération d'aérosol selon l'une quelconque des revendications précédentes, dans lequel le transducteur piézoélectrique (10) est un élément de génération d'aérosol configuré pour générer un aérosol à partir du substrat formant aérosol liquide.

8. Dispositif de génération d'aérosol selon la revendication 7, dans lequel le transducteur piézoélectrique (10) comprend une plaque perforée.

9. Dispositif de génération d'aérosol selon l'une quelconque des revendications précédentes, comprenant un réservoir de liquide (620) contenant un substrat formant aérosol liquide, dans lequel, en utilisation, le transducteur piézoélectrique est en contact avec le liquide provenant du réservoir de liquide.

10. Dispositif de génération d'aérosol selon la revendication 9, dans lequel le liquide comprend un mélange de différents composés.

11. Dispositif de génération d'aérosol selon la revendication 9 ou 10, dans lequel la circuiterie de commande (14) est configurée pour détecter une réduction de la quantité de liquide en contact avec le transducteur piézoélectrique sur la base de changements dans le comportement de résonance du transducteur piézoélectrique.

12. Dispositif de génération d'aérosol selon l'une quelconque des revendications précédentes, dans lequel le dispositif de génération d'aérosol est une cigarette électronique.

13. Dispositif de génération d'aérosol selon l'une quelconque des revendications précédentes, dans lequel le courant oscillant comprend une première fréquence modulée avec au moins une autre fréquence.

14. Procédé de fonctionnement d'un dispositif de génération d'aérosol le dispositif comprenant un ensemble transducteur dans une pompe à liquide, dans lequel l'ensemble transducteur comprend un transducteur piézoélectrique (10), une membrane ou une surface configurée pour entrer en contact avec un substrat formant aérosol liquide, le transducteur piézoélectrique étant configuré pour exciter la membrane ou la surface pour la mettre en vibration, la vibration de la membrane ou de la surface forçant le liquide à passer à travers une vanne de liquide adjacente dans la pompe à liquide, la circuiterie d'attaque (12) étant raccordée au transducteur piézoélectrique et la circuiterie de commande (14) étant configurée pour surveiller un paramètre du transducteur piézoélectrique et raccordée à la circuiterie d'attaque, le procédé comprenant :
l'application d'un courant oscillant au transducteur à l'aide de la circuiterie d'attaque ; et
la surveillance du comportement de résonance du transducteur piézoélectrique à l'aide de la circuiterie de commande, et
la commande du fonctionnement de la circuiterie d'attaque sur la base du comportement de résonance surveillé du transducteur piézoélectrique.
